# EUROPEAN PATENT SPECIFICATION

(11) **EP 4 288 563 B1**
(45) Date of publication and mention of the grant of the patent: **26.03.2025**
(21) Application number: 22707331.9
(22) Date of filing: 07.02.2022
(51) Int. Cl.: C12Q 1/6806

(54) **LIQUID COMPOSITION FOR MOLECULAR DIAGNOSTICS BY PCR**
FLÜSSIGE ZUSAMMENSETZUNG FÜR DIE MOLEKULARE DIAGNOSTIK MITTELS PCR
COMPOSITION LIQUIDE POUR DIAGNOSTIC MOLÉCULAIRE PAR PCR

(30) Priority: 08.02.2021 CZ 20205153
(43) Date of publication of application: 13.12.2023
(73) Proprietor: Bioinova, a.s., 14200 Praha 4 (CZ)
(72) Inventor: BAUER, Peter, 14300 Praha 4 (CZ); GUJSKI, Jana, 10100 Praha 10 (CZ); MATOSKA, Vaclav, 15200 Praha 5 (CZ)
(74) Representative: Harber IP s.r.o.
(86) International application number: PCT/CZ2022/050014
(87) International publication number: WO 2022/167017

(56) References cited:
- CA-A1- 2 901 406
- CN-A- 109 371 121
- CN-A- 110 257 534
- US-A1- 2003 078 412
- EL MAAIDEN EZZOUHRA ET AL: "Improved Method for DNA Extraction and Purification from Tetrahymena pyriformis", METHODS AND PROTOCOLS, vol. 2, no. 2, 15 May 2019 (2019-05-15), pages 40, XP055921286, DOI: 10.3390/mps2020040

## Description

### Field of Art

The invention relates to liquid compositions enabling decomposition of microorganisms while releasing and conserving nucleic acids. The thus isolated nucleic acids can be further detected or quantified by PCR.

### Background Art

Currently, quantitative PCR is a widely used method for the molecular diagnosis of infectious diseases caused by viral, bacterial or fungal pathogens. The fundamental condition for the use of this or any other method based on the detection of nucleic acids is their release from the viral capsid or microbial cell. Various methods for decomposing the capsids or cell walls/membranes of the pathogens are known, but most require additional steps of isolation or purification of the released RNA or DNA. It is essential to remove chemical compounds from the sample that potentially or provably inhibit subsequent enzymatic reactions, including PCR. Chemicals capable of decomposing or deactivating viral particles or cellular walls/membranes often also inactivate enzymes that are essential for successful PCR reaction. The need to remove such chemicals (eg thiocyanate) by more or less laborious methods of RNA/DNA isolation prolongs the time of the whole process of molecular diagnostics and, due to the use of isolation kits, also increases the costs. Moreover, such undesirable chemicals may not always be completely removed from the sample, which may result in a false negative test result and the need to repeat the RNA or DNA isolation. The isolation or purification methods are usually based on adsorption/elution and precipitation protocols. These procedures cause quantitative loss and reduced nucleic acid quality.

The use of ionic detergents, such as sodium dodecyl sulfate, for disruption of viral capsid or cell walls/membranes is known. Ionic detergents are widely used for RNA/DNA extraction, they are easily available and gentle. However, ionic detergents inhibit downstream enzymatic reactions at even relatively low concentrations, requiring their removal to neutralize their inhibitory effects. It has been proposed that non-ionic detergent may enhance the effect of ionic detergents in RNA/DNA extraction, however, no widely usable solution has been proposed to use the advantage of the ionic detergents but at the same time effectively eliminate their inhibitory effects on enzymatic reactions.

Further inhibitory substances may be present in the sample and thus transferred to the enzymatic reaction mix. Notably, such substances are metals. Liquid compositions for RNA/DNA extraction thus often contain chelators (EP 3636769, EP 1399459) or precipitation salts such as potassium hydroxide (CN 111139313). However, removal of chelators is necessary because they interfere with PCR reactions, thus requiring further purification steps. Moreover, potassium salts precipitate ionic detergents (such as SDS), and thus decrease their effects on viral capsids and microbial cell walls decomposition efficiency.

CA 2 901 406 A1 discloses a DNA extraction reagent, wherein the reagent comprises i) an alkali selected from the group consisting of potassium hydroxide, (KOH), metal hydroxide, alkali salts, and sodium hydroxide (NaOH), and combinations thereof; ii) a surfactant selected from the group consisting of SDS, Triton X-100, Triton X- 114, NP-40, Tween 20, Tween 80, Octyl glucoside, Octyl thioglucoside, CHAPS, and combinations thereof; iii) a salt selected from the group consisting of sodium chloride (NaCl), potassium chloride (KCl), potassium phosphates, sodium bicarbonate (NaHCO3), sodium acetate (C2H3NaO2), and ammonium acetate (NH4C2H3O2), and combinations thereof; and wherein the pH of the DNA extraction reagent is at least 10.

Therefore, it is necessary to develop liquid compositions which would maximize nucleic acid extraction , thus maximizing the PCR sensitivity, avoid additional intermediate purification steps causing quantitative nucleic acid loss and nucleic acid quality reduction while preventing inhibition of the PCR reactions.

### Disclosure of the Invention

The invention aims at providing a liquid composition which allows extraction of nucleic acids from the capsids or cells of the pathogens. The pathogens are microorganisms, typically viruses, bacteria or fungi. The liquid composition of the invention is universal, allowing the disruption of viral capsids as well as cell walls/membranes. The components of the composition are easily available and cost-effective.

The liquid composition of the invention extracts nucleic acids from the pathogen capsids or cells (i.e., for example viral capsids, bacterial cells and/or fungal cells), and the extracted sample can be used directly in a subsequent (downstream) PCR. Optionally, the extracted sample may be subjected to a short centrifugation if a visible turbidity due to the presence of biological material appears in the extracted sample. The use of the liquid composition of the invention is less instrumentally demanding (does not require specific equipment and material for RNA/DNA isolotaion and purification), as well as safer for the laboratory personnel (the pathogens are spontaneously inactivated). Such composition may advantageously be used for POC (point-of-care) assays.

The liquid composition of the invention contains 0.01-0.1 vol.% of at least one ionic detergent, 1-4 vol.% of at least one non-ionic detergent, and Na₃PO₄ and/or NaHCO₃.

The liquid composition preferably contains water, more preferably deionized water, double distilled water or water for injections, as a solvent. These types of treated water are sterile, free of nucleases and free of nucleic acids.

Ionic detergents include in particular sodium dodecyl sulfate (SDS), lauryl sulfate, deoxycholate, cholate and sarcosyl. SDS is particularly preferrred.

A preferred concentration of the ionic detergent in the liquid composition is within the range of 0.02-0.1 vol.%, preferably 0.02-0.05 vol.%, more preferably 0.02-0.03 vol.%, particularly preferably about 0.025 vol.%.

In some embodiments, particularly preferred ionic detergent is sodium dodecyl sulfate (SDS), wherein the concentration of SDS in the liquid composition is within the range of 0.01-0.05 vol.%, more preferably 0.02-0.03 vol.%, particularly preferably about 0.025 vol.%.

Non-ionic detergents preferably include Tweens (eg Tween-20 (polyoxyethylene sorbitan monolaurate), Tween-80 (polyoxyethylene sorbitan monooleate)), Triton X-100 (2-[4-(2,4,4-trimethylpentan-2-yl)phenoxy]ethanol), NP-40 (Igepal) or Nonidet P (octylphenoxypolyethoxyethanol). Tween 20 is particularly preferred.

A preferred concentration of the non-ionic detergent in the liquid composition is within the range of 2-4 vol.%, more preferably 2.5-3.5 vol.%, particularly preferably about 3 vol.%.

In some embodiments, particularly preferred non-ionic detergent is Tween 20, wherein the concentration of Tween 20 in the liquid composition is within the range of 2-4 vol.%, more preferably 2.5-3.5 vol.%, particularly preferably about 3 vol.%.

The ionic detergent disrupts viral particles / cell walls / cell membranes, and the nucleic acids are released into the solution. The solution also serves as an efficiont preservative for the released nucleic acids. The sample is thus usable for PCR reaction for up to several days after the nucleic acid extraction even when stored under normal temperature conditions (4-25 °C). It is thus not necessary to store the sample in demanding conditions, such as extreme cold which is usually used for storage of RNA in non-protecting enviroments. Freeze-thaw cycles may also cause deterioration of the nucleic acids. However, ionic detergents inhibit enzymatic reactions. In prior art procedures the ionic detergent must be removed from the solution, either by precipitation with mineral salts or by other process steps, which increase the financial and laboratory complexity of the prior art procedures. Moreover, these steps can also reduce the yield of the extracted nucleic acids and consequently reduce the sensitivity of the analysis causing eg. false negative results.

The addition of a nonionic detergent increases the effectiveness of the ionic detergents in nucleic acid extraction. Therefore, it is possible to reduce the concentration of the ionic detergent in the composition to a value at which its inhibitory effect on the downstream PCR is reduced to such an extent that the reaction can be performed with sufficient sensitivity without the need to remove the ionic detergent from the composition by precipitation with potassium, calcium or other salts.

The advantages of using a mixture of ionic and non-ionic detergents for processing the biological material (source of nucleic acids) include simplification and streamlining of the procedure, elimination of excessive contact of personnel with infectious biological material, reduction of financial and process requirements, better yields of nucleic acids and thus an increased robustness and reliability of the processes, including downstream processes.

Sodium dodecyl sulfate (SDS) is normally used as an ionic detergent in molecular applications at concentrations ranging from tenths of percent to several percents by volume. Equilibrium and arrested flow kinetic data indicate that unfolding of the tertiary structure of proteins at the SDS submicellar interface and chain expansion at the SDS micellar interface are the two main mechanisms in disrupting the protein structure. Protein chain expansion at micellar SDS concentration is controlled by coulombic repulsion between protein-bound micelles. Micelles in turn react with the side chains of anionic amino acids. The nature of the detergent-protein interaction is predominantly hydrophobic at submicellar and exclusively hydrophobic at micellar SDS concentrations. SDS is also able to react with highly denatured and negatively charged proteins, thus its interaction is independent of the structure, conformation and ionization state of the protein. However, for downstream enzymatic reactions, it is desirable to use the lowest possible concentration of SDS to avoid the risk of denaturation of the enzymes and of inhibition of the downstream enzymatic reaction.

Within the framework of the present invention, it was experimentally found that the ionic detergent SDS present in the liquid composition at concentrations higher than 0.1 vol.% significantly inhibits the downstream qPCR reaction. Although precipitation steps with potassium, calcium or other salts would lead to a partial removal of SDS from the solution, the sensitive enzymatic reactions, such as qPCR would still be inhibited, at least partly due to the salts dissolved in the solution.

To achieve the lowest possible concentration of ionic detergent capable of denaturing proteins and disrupting protein-protein interactions, a non-ionic detergent having the ability to disrupt lipid-lipid and protein-lipid interactions is present in the composition. However, unlike ionic detergents, the non-ionic detergents have a limited effect on protein denaturation and protein-protein interactions. Unlike ionic detergents, which react with oppositely charged regions of protein, the association of nonionic detergents with proteins is based purely on hydrophobic interactions. In the composition of the present invention, the denaturation process is thus initiated by the electrostatic interaction of the ionic detergent with the protein, disrupting its structue and exposing its hydrophobic regions, which then react with the non-ionic detergent(s).

Addition of non-ionic detergents to the liquid composition allowed the use a lower concentration of the ionic detergent while increasing the capsid/cell disintegration efficiency.

The liquid composition further contains Na₃PO₄ and/or NaHCO₃. These compounds allow to precipitate Ca²⁺ ions which are typically present in the biological materials including or originating from saliva, blood, nasopharynx, bone, etc. The Ca²⁺ ions precipitate the ionic detergents, in particular SDS, and thus would decrease the effectiveness of the liquid composition in extracting nucleic acids. At the low concentrations of the ionic detergents used in the present invention, it is necessary to use Na₃PO₄ and/or NaHCO₃ to maintain the efficiency in nucleic acid extraction while benefiting from the low ionic detergent content.

The concentration of Na₃PO₄ and/or NaHCO₃ in the liquid composition is preferably within the range of 0.1-100 mM, more preferably 0.1-30 mM, yet more preferably 0.5-20 mM., even more preferably 5-15 mM most preferably about 10 mM.

A further aspect of the present invention is a sampling kit for extraction of nucleic acids from pathogens in biological samples, which comprises a closable vial containing the liquid composition of the invention, and optionally a sampling swab. The vial may be provided with a removable screw cap. Such sampling kit may be used for collection of biological material such as swabs from the nasopharynx, neck, ear, skin or vaginal swabs, as well as swabs from the environment/surfaces that need to be monitored for pathogens, eg, in schools, hospitals, etc. Such sampling kit streamlines the processing of biological samples since the sample may be collected into the vial, nucleic acids are extracted in the same vial and the extracted nucleic acids can be stored for several days still in the same vial. Alternatively to storage or subsequently after storage, the extracted nucleic acids may be analyzed in the same vial by adding the reagents needed for perfoming a PCR reaction and performing the PCR reaction.

Yet further aspect of the invention is a method for extracting nucleic acids from a pathogen wherein the pathogen (or a biological sample containing the pathogen) is contacted with the liquid composition of the invention for a time necessary to disrupt the capsid or cell of the pathogen.

Yet another aspect of the invention is a method for detecting and/or quantifying the amount of a pathogen in a biological sample, which comprises the steps of
- contacting the biological sample with the liquid composition of the invention to extract nucleic acid(s) from the pathogen,
- optionally storing the extracted nucleic acid(s) in the liquid composition of the invention, and
- subsequently performing a PCR or quantitative PCR reaction to determine the presence and/or amount of the extracted pathogen nucleic acid(s).

The term "microorganism" or "pathogen" herein includes a virus, a bacterium or a fungus.

The term "nucleic acid" includes DNA and RNA.

As described above, it was surprisingly found that a very low amount of ionic detergent at concentrations not inhibiting downstream PCR is sufficient for capsid degradation or microbial cell wall degradation when combined with low concentration of non-ionic detergents. Moreover, a surprising effect of the presence of two salts in the liquid composition of the invention on enhancing the PCR sensitivity when analysing biological material containing divalent metal ions (such as calcium) was also identified. Therefore, a liquid composition and corresponding methods involving pathogen decomposition and deactivation and spontaneous isolation of nucleic acids are provided.

### Brief description of Drawings

Figure 1 shows the amplification curves obtained with samples listed in Table 2 (Example 2).
Figure 2 shows the amplification curves obtained with samples listed in Table 5 (Example 4).
Figure 3 shows an example plot of HCoV NL63 qPCR analysis (Example 6).
Figure 4 shows an example plot of HRSV qPCR analysis (Example 6).
Figure 5 shows the melting temperature curves obtained with samples listed in Table 9 (Example 7).

### Examples of carrying out the Invention

The examples shown herein serve to further illustrate the invention. The examples should not be construed as limiting the scope of the invention which is determined solely by the appended claims.

All PCR analyses in the examples shown in this chapter were performed with samples of SARS-CoV-2, using the GeneProof SARS-CoV-2 Screening PCR Kit (GeneProof, Czech Republic), unless specified otherwise. The PCR assay was carried out according to the manufacturer's instructions.

The liquid composition of the invention was also successfully tested using SARS-CoV-2 as well as other viruses, and using PCR kits from various manufacturers, including GeneProof, Generi Biotech, Elisabeth Pharmacon (all Czech Republic), BAG Diagnostics (Germany), iNtRON Biotechnology (Korea), Shanghai ZJ Bio-Tech (China) and PreciGenome (USA). It was also successfully evaluated on a LAMP test (Aumed, Czech Republic).

Contents of the detergents are shown in vol.% unless specified otherwise.

The water used in the examples was water for injections.

### Example 1 (Development Example)

The minimal effective ionic detergent concentration was determined based on experiments in which qPCR samples containing 1×10⁴ copies of SARS-CoV-2/ml were tested with 0, 0.01, 0.025, 0.05, and 0.1 vol.% SDS. KCl was added after 1 hr incubation at 24 °C to a final concentration of 200 mM to precipitate free SDS from the solution and to prevent its potential denaturing effect on enzymes in the quantitative RT-PCR. We have observed that SDS concentrations above 0.1 vol.% markedly inhibit the PCR reactions even after precipitation of free SDS by KCl.

To prepare the solutions, 20% SDS (Sigma-Aldrich) stock was used. To achieve required final concentrations, 0, 0.5, 1.25, 2.5 and 5 mL of the stock, respectively, was mixed with 1 L water. After adding the viruses to 1 mL aliquots and 1 hr incubation at 24 °C, KCl to final a concentration of 200 mM was added to the samples or were left without KCl. The samples were then analysed by qPCR using GeneProof SARS-CoV-2 PCR Kit according to the manufacturer's instructions and Rotor-Gene Q PCR cycler. Two genes were amplified - RNA-dependent RNA polymerase (FAM) and capsid gene N (TAMRA). For control of the presence of viral RNA, standard RNA isolation was also performed prior to qPCR analysis by Zybio Nucleic Acid Isolation System (Zybio, China). Results are shown in Table 1. All these SDS concentrations were found to have, inhibitory effects on the PCR when not precipitated. With no SDS, no signal could be observed without prior RNA isolation. Additionally, SDS concentration of 0.01 vol.% and lower lead to weak PCR signal, most probably due its level not able to efficiently decompose the viral particles while providing reduced yield of the RNA.

**Table 1. Comparison of different SDS concentrations and its precipitation by KCl in viral RNA PCR analysis (Y = Yes, N = No, Y/N = weak PCR signal; RNA isolation refers to the RNA isolation using the Zybio commercial kit).**

| | | **FAM** | | **TAMRA** | |
|---|---|---|---|---|---|
| **SDS concentration (%)** | **KCl (200 mM)** | **Positive signal** | **Positive signal after RNA isolation** | **Positive signal** | **Positive signal after RNA isolation** |
| 0 | Y | N | Y | N | Y |
| | N | N | Y | N | Y |
| 0.01 | Y | Y/N | Y | Y/N | Y |
| | N | N | Y | N | Y |
| 0.025 | Y | Y | Y | Y | Y |
| | N | N | Y | N | Y |
| 0.05 | Y | Y | Y | Y | Y |
| | N | N | Y | N | Y |
| 0.1 | Y | Y | Y | Y | Y |
| | N | N | Y | N | Y |

### Example 2 (Development Example)

Next, for the following tests, we focused on the low but efficient SDS concentration of 0.025 vol.%, where a non-ionic detergent is added to eliminate the inhibitory effect of SDS on PCR.

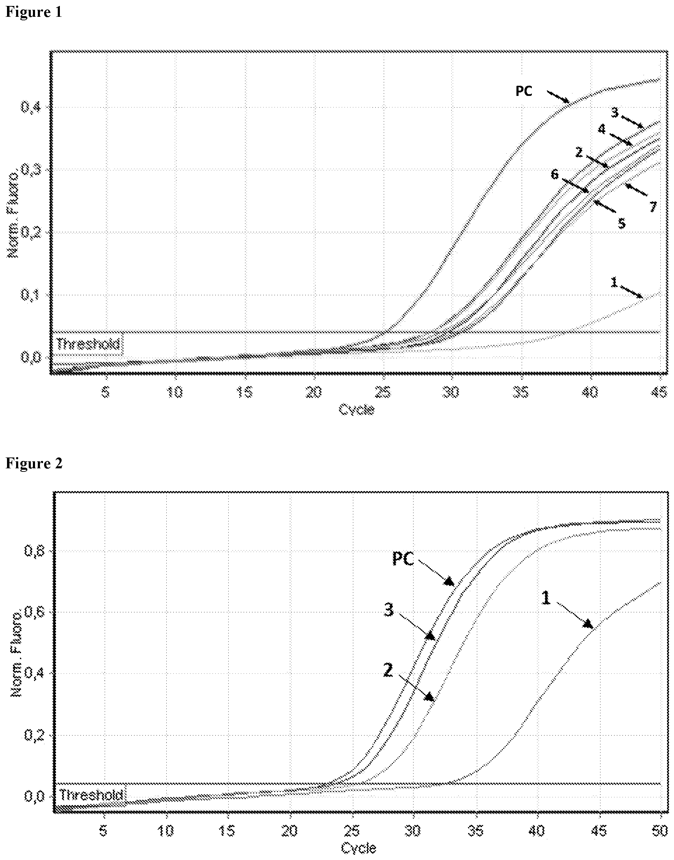

To the 0.025 vol.% SDS solution, 2, 3 or 4 vol.% Tween 20 or Triton X-100 was added, then precipitation by 200 mM KCl was performed. PCR was conducted as described above. The results (FAM channels) are shown in Table 2 and Figure 1. Surprisingly, such combination substantially increased the PCR performance. The best result was achieved by the combination of 0.025 vol.% SDS and 3 vol.% Tween-20 and this solution was used for further tests.

**Table 2. Evaluation of ionic detergent (SDS) and non-ionic detergent (Tween 20 or Triton X-100) solution.**

| **Sample** | **SDS (vol. %)** | **Tween20 (vol.%)** | **Triton X-100 (vol.%)** | **KCl (mM)** | **Cₜ** |
|---|---|---|---|---|---|
| Positive control RNA (PC) | 0.025 | - | - | 200 | 25,08 |
| 1 | 0.025 | - | - | 200 | 38.27 |
| 2 | 0.025 | 2 | - | 200 | 29.74 |
| 3 | 0.025 | 3 | - | 200 | 28.38 |
| 4 | 0.025 | 4 | - | 200 | 28.50 |
| 5 | 0.025 | - | 2 | 200 | 30.64 |
| 6 | 0.025 | - | 3 | 200 | 29.21 |
| 7 | 0.025 | - | 4 | 200 | 30.27 |

### Example 3

In this example, neutralization of inhibitory effects of metal ions (eg, Ca²⁺ ions) in biological samples were evaluated. Such ions have the potential to inhibit PCR eg. due to competition with essential cofactors, eg. magnesium.

Effects of two salts Na₃PO₄ and NaHCO₃ which are able to bind divalent ions often present in biological material are shown. These salts improve the performance of PCR, and there are surprisingly no undesired interactions with other components of the liquid composition, nor with PCR reagents. Moreover, these salts can maintain pH, thus enabling a longer storage life of the liquid composition. Otherwise, pH may decrease upon storage of the solution and can reduce the performance of the detergents.

Herein, we prepared 0.025 vol.% SDS / 3 vol.% Tween 20 solution and added Na₃PO₄ or NaHCO₃ at final concentrations of 0, 0.01, 0.1, 1, 5, 10, 15, 20, or 100 mM. The solutions were aliquoted to 1 mL samples and 100 µL of human saliva was added to each sample to mimic the presence of biological material containing Ca²⁺. To evaluate the potential inhibitory effects of saliva on the PCR efficiency, the reaction was performed using Positive control RNA supplied with the GeneProof SARS-CoV-2 PCR Kit. The results are summarized in Table 3 (Na₃PO₄) and Table 4 (NaHCO₃).

**Table 3. Evaluation of Na₃PO₄ in 0.025 vol.% SDS / 3 vol.% Tween 20 solution containing human saliva (avg = average).**

| Na₃PO₄ (mM) | RdRP gene (Ct) | | | | |
|---|---|---|---|---|---|
| | RNA | avg | RNA+saliva | avg | p value |
| 20 | 26.79 | 26.58 | 26.52 | 26.57 | 0.4805621 |
| | 26.63 | | 26.53 | | |
| | 26.32 | | 26.66 | | |
| 15 | 26.07 | 26.09 | 26.03 | 26.05 | 0.0638216 |
| | 26.16 | | 26.15 | | |
| | 26.03 | | 25.97 | | |
| 10 | 25.59 | 25.64 | 25.65 | 25.41 | 0.2059647 |
| | 25.84 | | 25.17 | | |
| | 25.48 | | 25.40 | | |
| 5 | 25.56 | 25.59 | 25.16 | 25.81 | 0.2918334 |
| | 25.26 | | 26.01 | | |
| | 25.96 | | 26.26 | | |
| 1 | 25.50 | 25.50 | 25.71 | 25.57 | 0.3100927 |
| | 25.68 | | 25.51 | | |
| | 25.32 | | 25.49 | | |
| 0.1 | 25.41 | 25.40 | 26.99 | 26.83 | 0.0028739 |
| | 25.42 | | 26.64 | | |
| | 25.36 | | 26.86 | | |
| 0.01 | 25.37 | 25.54 | 28.03 | 28.02 | 0.0011127 |
| | 25.59 | | 28.11 | | |
| | 25.67 | | 27.93 | | |
| 0 | 24.86 | 25.37 | 27.48 | 28.02 | 0.0008262 |
| | 25.75 | | 28.23 | | |
| | 25.51 | | 28.36 | | |

**Table 4. Evaluation of NaHCO₃ in 0.025 vol.% SDS / 3 vol.% Tween 20 solution containing human saliva.**

| NaHCO₃ (mM) | RdRP gene (Ct) | | | | |
|---|---|---|---|---|---|
| | RNA | avg | RNA+saliva | avg | p value |
| 100 | 26.57 | 26.59 | 27.22 | 27.33 | 0.0057726 |
| | 26.65 | | 27.32 | | |
| | 26.56 | | 27.46 | | |
| 20 | 25.91 | 25.76 | 26.74 | 26.68 | 0.0291556 |
| | 25.80 | | 26.37 | | |
| | 25.57 | | 26.93 | | |
| 15 | 25.48 | 25.64 | 25.7 | 25.98 | 0.0212059 |
| | 25.81 | | 26.14 | | |
| | 25.63 | | 26.1 | | |
| 10 | 25.49 | 25.43 | 25.81 | 25.99 | 0.0821671 |
| | 25.45 | | 25.73 | | |
| | 25.35 | | 26.43 | | |
| 5 | 25.72 | 25.73 | 26.23 | 26.06 | 0.0804142 |
| | 25.75 | | 26.19 | | |
| | 25.72 | | 25.75 | | |
| 1 | 25.37 | 25.41 | 25.89 | 25.82 | 0.0720282 |
| | 25.42 | | 25.49 | | |
| | 25.43 | | 26.09 | | |
| 0.1 | 25.58 | 25.51 | 26.62 | 26.84 | 0.0109392 |
| | 25.63 | | 26.88 | | |
| | 25.31 | | 27.03 | | |
| 0.01 | 25.34 | 25.41 | 27,84 | 27.30 | 0.0127765 |
| | 25.38 | | 27.04 | | |
| | 25.52 | | 27.03 | | |
| 0 | 25,27 | 25.44 | 28,43 | 28.14 | 0.0054602 |
| | 25.75 | | 28.23 | | |
| | 25.29 | | 28.36 | | |

These data clearly show the inhibition of PCR by saliva and indicate that Na₃PO₄ and NaHCO₃ prevent or decrease this inhibition. As the most efficient embodiment was found to be 10 mM Na₃PO₄, the composition of the liquid composition for further tests was set to 0.025 vol.% SDS, 3 vol.% Tween-20 and 10 mM Na₃PO₄.

### Example 4

In this example, effect of each component of the liquid composition was evaluated (SDS, Tween 20, Na₃PO₄) at most advantageous concentrations in the combination. Three solutions were prepared in water for injections:
1. 0.025 vol.% SDS
2. 0.025 vol.% SDS + 3 vol.% Tween 20
3. 0.025 vol.% SDS + 3 vol.% Tween 20 + 10 mM Na₃PO₄

After mixing, 1×10⁵ SARS-CoV-2 viral particles and 10 µL of human saliva were added to 1 mL aliquots and incubated for 1 hr at 24 °C. Then, the PCR analysis was performed as described above. Positive control RNA supplied with the GeneProof SARS-CoV-2 PCR Kit was used as positive control (PC). The results are summarized in Table 5 and shown in Figure 2. The data clearly show the synergic effects of Tween 20 and Na₃PO₄ in the SDS solution on the RNA recovery from the viral particles and the PCR efficiency and sensitivity.

**Table 5. Evaluation of the 0.025 vol.% SDS / 3 vol.% Tween 20 / 10 mM Na₃PO₄ solution containing human saliva on PCR analysis efficiency and sensitivity from SARS-CoV-2.**

| **Sample** | **Viral particles/mL** | **SDS (0.025 %)** | **Tween 20 (3 %)** | **Na₃PO₄ (10 mM)** | **Cₜ** |
|---|---|---|---|---|---|
| Positive control (PC) | 10⁵ | N | N | N | 22.91 |
| 1. | 10⁵ | Y | N | N | 32.14 |
| 2. | 10⁵ | Y | Y | N | 25.36 |
| 3. | 10⁵ | Y | Y | Y | 23.09 |

### Example 5

Due to the release of unstable RNA molecules from viral capsids to the solution, we evaluted its stability upon sample storage. The aim was to mimic the time which elapses between the sample collection and qPCR analysis. Such time delay may be caused by transportation from the sampling site to the laboratory or delay by a limited capacity of the diagnostic laboratory to process the samples, eg. due to large number of samples. We collected samples from five SARS-CoV-2-positive patients and stored them at 4°C or 24°C for 24, 48 and 72 hours. At each time point, qPCR analysis was performed by GeneProof SARS-CoV-2 PCR Kit according to the manufacturer's instructions. Surprisingly, during 72 hours storage at both tested temperatures, no reduction in qPCR yields was observed (Table 6). These results indicate that the solution is not only able to decompose the viruses and release the RNA, but also provides an environment conserving RNA for PCR analysis.

**Table 6. Evaluation of the clinical samples' stability (released RNA) in the 0.025 vol.% SDS / 3 vol.% Tween 20 / 10 mM Na3PO4 solution. Ct values for the RdRP gene (FAM channel) are shown.**

| **Sample No.** | **24 hrs** | | **48 hrs** | | **72 hrs** | |
|---|---|---|---|---|---|---|
| | **4°C** | **24°C** | **4°C** | **24°C** | **4°C** | **24°C** |
| 1 | 29.14 | 23.02 | 27.79 | 23.76 | 28.93 | 22.48 |
| 2 | 29.96 | 27.45 | 26.97 | 27.25 | 29.16 | 27.07 |
| 3 | 27.29 | 28.05 | 25.54 | 28.07 | 25.04 | 28.50 |
| 4 | 26.56 | 25.36 | 24.05 | 25.51 | 22.66 | 25.07 |
| 5 | 18.03 | 24.70 | 18.26 | 24.84 | 18.04 | 24.52 |

### Example 6

In this example, we tested the liquid composition for SARS-CoV-2 on other viruses. Swabs from 30 patients with clinical diagnosis of respiratory infections were collected to the 0.025 vol.% SDS / 3 vol.% Tween 20 / 10 mM Na₃PO₄ solution in water for injections. Half of the samples' volume was subjected to RNA isolation by Quick DNA/RNA Viral Kit (Zymo Research, USA). The isolated RNA and the intact samples were used for PCR analysis by FTlyo Respiratory pathogens 21 (Fast Track Diagnostics, Malta). List of the tested pathogens is shown in Table 7. In 14 patients, various infections were detected (influenza virus A/H1N1, human rhinovirus, human adenovirus, enterovirus and human parechovirus) (Table 8). In 16 patients, no viral infection was detected.

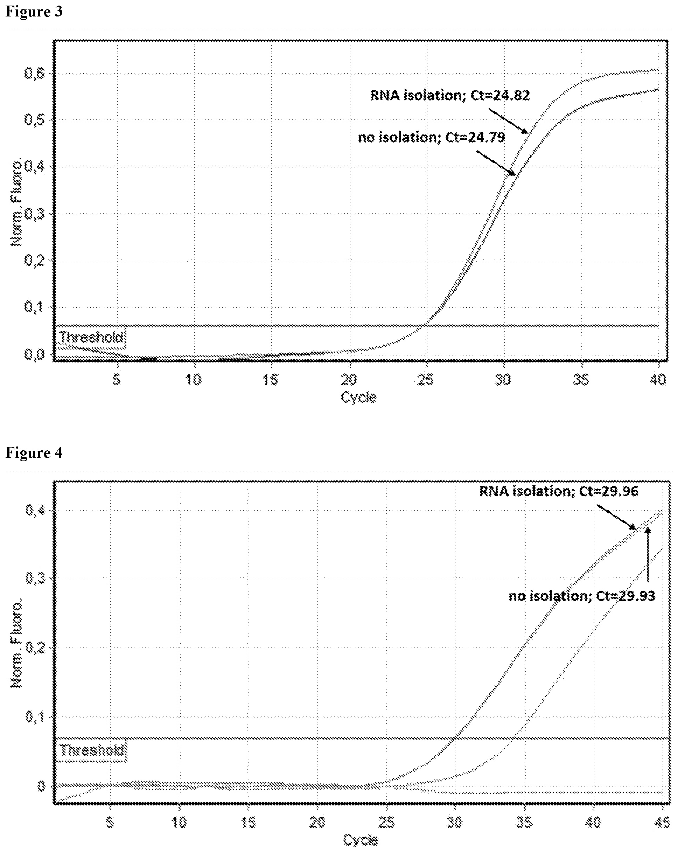

Surprisingly, there was no discrepancy between the isolated and non-isolated samples observed, both methods were able detect the presence of viruses with same efficiency and similar sensitivity (for illustration, see Fig. 3 - HCoV NL63 and Fig. 4 - HRSV). Moreover, a substantial time (time required for the RNA isolation) and cost reduction of the procedure was achieved when the liquid composition of the invention was used. This example shows that the composition of the invention is suitable for molecular diagnostics of various viral species, including DNA viruses (eg. HAdV).

**Table 7. Inclusivity of FTlyo Respiratory pathogens 21.**

| **Pathogens** | **Abbreviation** | **Subtypes Detected** |
|---|---|---|
| enterovirus | EV | Species A-D |
| human adenovirus | HAdV | All seven species of HAdV (A-G) |
| human bocavirus | HBoV | HBoV subtype 1 |
| human coronavirus 229E | HCoV 229E | No subtypes defined |
| human coronavirus HKU 1 | HCoV HKU1 | |
| human coronavirus NL63 | HCoV NL63 | |
| human coronavirus OC43 | HCoV OC43 | |
| human metapneumoviruses | HumpV | Genotypes A and B |
| human parainfluenza 1 virus | HPIV-1 | No subtypes defined |
| human parainfluenza 2 virus | HPIV-2 | |
| human parainfluenza 3 virus | HPIV-3 | |
| human parainfluenza 4 virus | HPIV-4 | |
| human parechovirus | HPeV | Subtypes 1-8, 10, 14 and 16-18 |
| human respiratory syncytial viruses | HRSV | Subtypes A and B |
| human rhinovirus | HRV | Species HRV A, B and C |
| influenza A virus | IAV | Serotypes H1-H16 |
| influenza A virus H1N1 swl | IAV (H1N1) swl | 2009-pandemic swine-lineage influenza A virus subtype H1N1 |
| influenza B virus | IBV | Yamagata and Victoria lineages |
| mycoplasma pneumoniae | M. pneumoniae | Subtypes 1 and 2 |

**Table 8. Detection of various viruses by FTlyo Respiratory pathogens 21 system.**

| | **Positivity (n)** | |
|---|---|---|
| **Pathogen** | **DNA/RNA isolation (comparative)** | **No DNA/RNA isolation - direct PCR - using the composition of the invention** |
| EV | 1 | 1 |
| HAdV | 3 | 3 |
| HBoV | | |
| HCoV 229E | 2 | 2 |
| HCoV HKU1 | 2 | 2 |
| HCoV NL63 | 1 | 1 |
| HCoV OC43 | | |
| HumpV | | |
| HPIV-1 | | |
| HPIV-2 | | |
| HPIV-3 | | |
| HPIV-4 | | |
| HPeV | 1 | 1 |
| HRSV | 1 | 1 |
| HRV | 1 | 1 |
| IAV | 1 | 1 |
| IAV (H1N1) swl | 1 | 1 |
| IBV | | |
| M. pneumoniae | | |

### Example 7

The composition of the present invention containing 0.025 vol.% SDS / 3 vol.% Tween 20 / 10 mM Na₃PO₄ solution in water for injections was tested in detection of VanA and VanB genes in Vancomycin-resistant *Enterococcus* (VRE) bacteria. 31 clinical samples were obtained from patients (abscesses, peritoneal fluid, throat mucus, or stool). In ten of them, VRE resistant Enterococcus faecium infection was previously found by cultivation. First, part of each biological sample (50 µL) was brought in contact with 1 mL of the 0.025 vol% SDS / 3 vol.% Tween 20 / 10 mM Na₃PO₄ solution - these were test samples. From a second part of each biological sample, DNA isolation was performed by Geneproof pathogene isolation kit according to the manufacturer's protocol - these were control samples. For qPCR, DNA isolates (control samples) as well as test samples were used, employing Geneproof VRE PCR kit that detects VanA and VanB sequences. The biological material contained the same amount of DNA for both types of samples - control sample and test sample (proportional volume of not-isolated samples in comparison to input material for DNA isolation was used). Out of 31 specimens, one VanA- and five VanB-positive samples were found. We found a surprising 100% concordance in detection of VanA or VanB genes between isolated bacterial DNA (control samples) and test samples using the composition of the present invention from biological samples of patients with suspected Vancomycin-resistant *E*. *faecium.* The results (positive and negative) for the corresponding control samples and test samples were the same. This indicates that the liquid composition of the present invention is able to release bacterial genetic material at levels sufficient for PCR assays and that the results are comparable to those provided by standard isolation of DNA.

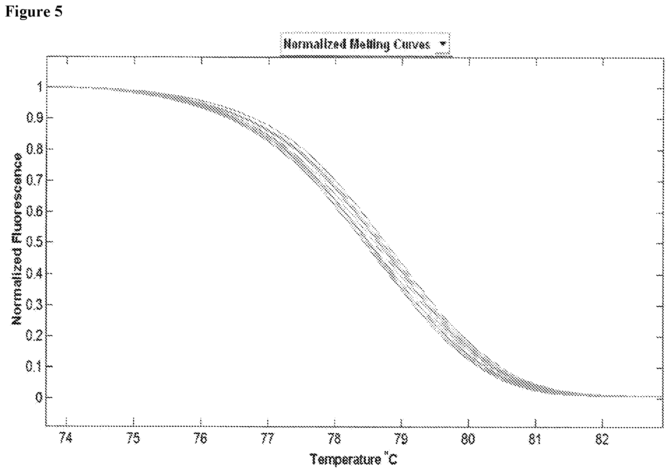

To compare the specific DNA yield from the samples prepared by both methods, high resolution melting analysis (HRM) was performed using using LightScanner mastermix containg LCGreen dye (Idahotech) and following primers: Vanabf: 5-CTG TTT GAA TTG TCC GGT ATC CC-3 (SEQ ID NO. 1) and Vanabr: 5-GAG CTT TGA ATA TCG CAG CCT AC-3 (SEQ ID NO. 2). The resulting reactions were then analyzed by LightScanner instrument. The median of the melting temperatures (Tm) was 78.59°C for the 49 bp PCR product (Table 9; standard error = 0.03). The intensity of the fluorescence from intercalated LCGreen dye showed no significant differences between the samples (Figure 5).

**Table 9. High resolution melting analysis.**

| | **Tm (°C)** | |
|---|---|---|
| **Sample** | **DNA isolation (comparative)** | **No DNA/RNA isolation - direct PCR - using the composition of the invention** |
| 1 | 78.44 | 78.50 |
| 2 | 78.46 | 78.66 |
| 3 | 78.52 | 78.66 |
| 4 | 78.51 | 78.72 |
| 5 | 78.60 | 78.78 |

### Example 8: Liquid compositions

The following liquid compositions are prepared by preparing a solution of the ionic detergent and the non-ionic detergent in water for injections and adding Na₃PO₄ and/or NaHCO₃ to the indicated final concentrations (Table 10).

**Table 10.**

| | **ionic detergent SDS (vol. %)** | **non-ionic detergent (type, vol. %)** | **Na₃PO₄ (mM)** | **NaHCO₃ (mM)** |
|---|---|---|---|---|
| Example A | 0.01 | Tween20, 2 | 0.5 | --- |
| Example B | 0.025 | TritonX-100, 3 | 5 | --- |
| Example C | 0.05 | TritonX-100, 3 | --- | 30 |
| Example D | 0.1 | Tween20, 4 | 15 | 5 |
| Example E | 0.025 | Tween20, 3 | 5 | 5 |
| Example F | 0.025 | Tween20, 2.5 | 10 | --- |
| Example G | 0.025 | Tween20, 3 | --- | 1 |

## Claims

1. Liquid composition for extracting nucleic acid(s) from pathogens, said liquid composition containing 0.01-0.05 vol.% of at least one ionic detergent, 1-4 vol.% of at least one non-ionic detergent, and Na₃PO₄ and/or NaHCO₃.

2. The liquid composition according to claim 1, which further contains water, preferably deionized water, double distilled water or water for injections, as a solvent.

3. The liquid composition according to claim 1 or 2, wherein the ionic detergent is sodium dodecyl sulfate.

4. The liquid composition according to any one of the preceding claims, wherein the concentration of the ionic detergent in the liquid composition is within the range of 0.02-0.03 vol.%.

5. The liquid composition according to any one of the preceding claims, wherein the non-ionic detergent is Tween-20.

6. The liquid composition according to any one of the preceding claims, wherein the concentration of the non-ionic detergent in the liquid composition is within the range of 2-4 vol.%, preferably 2.5-3.5 vol.%.

7. The liquid composition according to any one of the preceding claims, wherein the concentration of Na₃PO₄ and/or NaHCO₃ in the liquid composition is within the range of 0.1-100 mM, more preferably 0.5-20 mM.

8. The liquid composition according to claim 1, said liquid composition containing 0.01-0.05 vol.% of sodium dodecyl sulfate, 1-4 vol.% of Tween 20, and 0.1-100 mM Na₃PO₄ and/or NaHCO₃.

9. The liquid composition according to claim 1, said liquid composition containing 0.01-0.05 vol.% of sodium dodecyl sulfate, 1-4 vol.% of Tween 20, and 0.1-100 mM Na₃PO₄.

10. A sampling kit for extraction of nucleic acids from pathogens in biological samples, which comprises a closable vial containing the liquid composition according to any one of the preceding claims, and optionally a sampling swab in an aseptic packaging.

11. A method for extracting nucleic acids from a pathogen, wherein the pathogen or a biological sample containing the pathogen is contacted with the liquid composition according to any one of claims 1 to 9 for a time necessary to disrupt the capsid or cell of the pathogen.

12. A method for detecting and/or quantifying the amount of a pathogen in a biological sample, which comprises the steps of
- contacting the biological sample with the liquid composition according to any one of claims 1 to 9 to extract nucleic acid(s) from the pathogen,
- optionally storing the extracted nucleic acid(s) in the liquid composition according to any one of claims 1 to 9, and
- subsequently performing a PCR or quantitative PCR reaction to determine the presence and/or amount of the extracted pathogen nucleic acid(s).

## Patentansprüche

1. Flüssige Zusammensetzung zum Extrahieren von Nukleinsäure(n) aus Krankheitserregern, wobei die flüssige Zusammensetzung 0,01-0,05 Vol.-% von mindestens einem ionischen Detergens, 1-4 Vol.-% von mindestens einem nichtionischen Detergens, und Na₃PO₄ und/oder NaHCO₃ enthält.

2. Flüssige Zusammensetzung nach Anspruch 1, die zusätzlich Wasser, vorzugsweise deionisiertes Wasser, doppelt destilliertes Wasser oder Wasser für Injektionszwecke, als Lösungsmittel enthält.

3. Flüssige Zusammensetzung nach Anspruch 1 oder 2, wobei das ionische Detergens Natriumdodecylsulfat ist.

4. Flüssige Zusammensetzung nach einem der vorhergehenden Ansprüche, wobei die Konzentration des ionischen Detergens in der flüssigen Zusammensetzung im Bereich von 0,02-0,03 Vol.-% liegt.

5. Flüssige Zusammensetzung nach einem der vorhergehenden Ansprüche, wobei das nichtionische Detergens Tween-20 ist.

6. Flüssige Zusammensetzung nach einem der vorhergehenden Ansprüche, wobei die Konzentration des nichtionischen Detergens in der flüssigen Zusammensetzung im Bereich von 2-4 Vol.-%, vorzugsweise 2,5-3,5 Vol.-% liegt.

7. Flüssige Zusammensetzung nach einem der vorhergehenden Ansprüche, wobei die Konzentration von Na₃PO₄ und/oder NaHCO₃ in der flüssigen Zusammensetzung im Bereich von 0,1-100 mM, vorzugsweise 0,5-20 mM liegt.

8. Flüssige Zusammensetzung nach Anspruch 1, wobei die flüssige Zusammensetzung 0,01-0,05 Vol.- % Natriumdodecylsulfat, 1-4 Vol.-% Tween 20 und 0,1-100 mM Na₃PO₄ und/oder NaHCO₃ enthält.

9. Flüssige Zusammensetzung gemäß Anspruch 1, wobei die flüssige Zusammensetzung 0,01-0,05 Vol.-% Natriumdodecylsulfat, 1-4 Vol.-% Tween 20 und 0,1-100 mM Na₃PO₄ enthält.

10. Probenentnahmekit zur Extraktion von Nukleinsäuren aus Krankheitserregern in biologischen Proben, das eine verschließbare Phiole mit der flüssigen Zusammensetzung nach einem der vorhergehenden Ansprüche und optional einen Probenentnahmetupfer in einer aseptischen Verpackung umfasst.

11. Verfahren zur Extraktion von Nukleinsäuren aus einem Krankheitserreger, wobei der Krankheitserreger oder eine biologische Probe, die den Krankheitserreger enthält, mit der flüssigen Zusammensetzung nach einem der Ansprüche 1 bis 9 in Kontakt gebracht wird für eine Zeit, die erforderlich ist, um die Kapside oder Zelle des Krankheitserregers aufzubrechen.

12. Verfahren zum Nachweis und/oder zur Quantifizierung der Menge eines Krankheitserregers in einer biologischen Probe, das die Schritte umfasst:
- Inkontaktbringen der biologischen Probe mit der flüssigen Zusammensetzung nach einem der Ansprüche 1 bis 9, um Nukleinsäure(n) aus dem Krankheitserreger zu extrahieren,
- optionales Aufbewahren der extrahierten Nukleinsäure(n) in der flüssigen Zusammensetzung nach einem der Ansprüche 1 bis 9, und
- anschließendes Durchführen einer PCR- oder quantitativen PCR-Reaktion, um das Vorhandensein und/oder die Menge der extrahierten Krankheitserreger-Nukleinsäure(n) zu bestimmen.

## Revendications

1. Composition liquide pour extraire un ou plusieurs acides nucléiques à partir d'un pathogène, ladite composition liquide contenant 0,01 à 0,05 % en volume d'au moins un détergent ionique, 1 à 4 % en volume d'au moins un détergent non ionique, et Na₃PO₄ et/ou NaHCO₃.

2. Composition liquide selon la revendication 1, qui contient en outre de l'eau, de préférence de l'eau déionisée, de l'eau bidistillée ou de l'eau pour préparations injectables, comme solvant.

3. Composition liquide selon la revendication 1 ou 2, où le détergent ionique est le dodécyl sulfate de sodium.

4. Composition liquide selon l'une quelconque des revendications précédentes, où la concentration du détergent ionique dans la composition liquide est comprise entre 0,02 et 0,03 % en volume.

5. Composition liquide selon l'une quelconque des revendications précédentes, où le détergent non ionique est le Tween-20.

6. Composition liquide selon l'une quelconque des revendications précédentes, où la concentration du détergent non ionique dans la composition liquide est comprise entre 2 et 4 % en volume, de préférence entre 2,5 et 3,5 % en volume.

7. Composition liquide selon l'une quelconque des revendications précédentes, où la concentration de Na₃PO₄ et/ou NaHCO₃ dans la composition liquide est comprise entre 0,1 et 100 mM, de préférence entre 0,5 et 20 mM.

8. Composition liquide selon la revendication 1, ladite composition liquide contenant 0,01 à 0,05 % en volume de dodécyl sulfate de sodium, 1 à 4 % en volume de Tween 20, et 0,1 à 100 mM de Na₃PO₄ et/ou NaHCO₃.

9. Composition liquide selon la revendication 1, ladite composition liquide contenant 0,01 à 0,05 % en volume de dodécyl sulfate de sodium, 1 à 4 % en volume de Tween 20, et 0,1 à 100 mM de Na₃PO₄.

10. Kit d'échantillonnage pour l'extraction d'acides nucléiques à partir d'un pathogène dans des échantillons biologiques, qui comprend un flacon refermable contenant la composition liquide selon l'une quelconque des revendications précédentes, et éventuellement un tampon d'échantillonnage dans un emballage aseptique.

11. Procédé d'extraction d'acides nucléiques à partir d'un pathogène, où le pathogène ou un échantillon biologique contenant le pathogène est mis en contact avec la composition liquide selon l'une quelconque des revendications 1 à 9 pendant une durée nécessaire pour rompre la capside ou la cellule du pathogène.

12. Procédé de détection et/ou de quantification de la quantité d'un pathogène dans un échantillon biologique, qui comprend les étapes consistant à
- mettre en contact l'échantillon biologique avec la composition liquide selon l'une quelconque des revendications 1 à 9 pour extraire le ou les acide(s) nucléique(s) du pathogène,
- éventuellement stocker le ou les acide(s) nucléique(s) extraits dans la composition liquide selon l'une quelconque des revendications 1 à 9, et
- effectuer ensuite une réaction de PCR ou de PCR quantitative pour déterminer la présence et/ou la quantité du ou des acide(s) nucléique(s) extraits du pathogène.
